# EUROPEAN PATENT APPLICATION

(11) **EP 4 295 842 A1**
(43) Date of publication of application: **27.12.2023**
(21) Application number: 22181025.2
(22) Date of filing: 24.06.2022
(51) Int. Cl.: A61K 31/19, A61K 31/17, A61K 31/714, A61P 17/06, A61K 9/00

(54) **A SKIN CARE COMPOSITION**

(71) Applicant: Faaborg Pharma - IP ApS, 5600 Faaborg (DK)
(72) Inventor: NIELSEN, Sten Grønved, 5600 Faaborg (DK)
(74) Representative: COPA Copenhagen Patents

(57) **Abstract**

The present disclosure relates to a skin care composition comprising boswellic acids and vitamin B12 or derivatives thereof. The compositions can be advantageously used in a method of treatment of dermatological conditions, disorders or diseases, in particular psoriasis, more particularly plaque psoriasis.

## Description

The present disclosure relates to a skin care composition comprising boswellic acids and vitamin B12 or derivatives thereof. The compositions of the present disclosure can be advantageously used in a method of treatment of dermatological conditions, disorders, or diseases, in particular psoriasis, in particular plaque psoriasis.

### BACKGROUND

Psoriasis is a chronic, non-infectious, inflammatory disease of the skin characterized by areas of abnormally raised skin. These areas may be red or purple, dry, itchy, and usually contain plaques with a silvery-white scaling. The underlying disease mechanism involves the immune system reacting to skin cells. Psoriasis varies in severity from small, localized patches to complete body coverage. Psoriasis is generally thought to be a genetic disease that is triggered by environmental factors, and about one third of the affected patients are affected due to genetic factors. The disease can further be provoked or aggravated by environmental factors such as infections, exposure to UV radiation, stress, artificial food products, alcohol, smoking, or various drugs. Sometimes, psoriasis is accompanied by joint disorders or autoimmune conditions. Symptoms often worsen during winter and with the simultaneous use of certain medications, such as beta blockers or NSAIDs. The disease affects 2-4% of the population and the disease may begin at any age, but typically starts in adulthood. Psoriasis is associated with an increased risk of psoriatic arthritis, lymphomas, cardiovascular disease, Crohn's disease, and depression. In particular, many patients suffering from severe psoriasis experience invalidation itching and discomfort and experience a significant reduction in their quality of life. Psoriatic arthritis affects up to 30% of individuals with psoriasis.

The main types of psoriasis are plaque, guttate, inverse, pustular, and erythrodermic psoriasis. Plaque psoriasis, also known as psoriasis vulgaris, makes up about 90% of cases. It typically presents as red patches with white scales on top. Areas of the body most commonly affected are the back of the forearms, shins, navel area, and scalp.

There is no known cure for psoriasis, but various treatments can help control the symptoms. These treatments include steroid creams, vitamin D3 cream, ultraviolet light, and immunosuppressive drugs, such as methotrexate. More severe forms of the disease often have to be treated systemically using photochemical therapies, retinoids, methotrexate, hydroxyurea, azathioprine or cyclosporin. However, some of these treatments have severe side effects and/or are not effective in some patients. Further, treatments of more severe forms of psoriasis are costly, and often show undesirable side effects, and have a varying efficacy.

There is therefore a need in the art for more effective topical treatment alternatives.

Accordingly, the technical problem underlying the present invention is to provide improved topical compositions for the treatment of psoriasis, in particular to provide improved topical compositions for the treatment of plaque psoriasis. These means should be efficient and lead to a substantial amelioration of the symptoms and shall have no or only mild side effects.

The solution to the above technical problem is achieved by the compositions according to the present invention.

Vitamin B12 (cyanocobalamin or cobalamin) is a water-soluble vitamin involved in DNA synthesis and in both fatty acid and amino acid metabolism. It is important in the normal functioning of the nervous system via its role in the synthesis of myelin, and in the circulatory system in the maturation of red blood cells in the bone marrow. The term "vitamin B12 or a derivative thereof" as used herein includes all forms of vitamin B12 and any compounds that have vitamin B12 activity. In particular, said term includes cobalamin, hydroxycobalamin, cyanocobalamin, methylcobalamin, adenosylcobalamin, 5'-deoxyadenosyl-cobalamin, aquocobalamin, and nitritocobalamin.

Boswellic acids are organic acids consisting of a pentacyclic triterpene, a carboxyl group and at least one other functional group. Alpha-boswellic acid and beta-boswellic acid (C₃₀H₄₈O₃) both have an additional hydroxyl group and differ only in their triterpene structure. In acetyl-alpha-boswellic acid and acetyl-beta-boswellic acid (C₃₂H₅₀O₄), a hydroxyl group is replaced by an acetyl group. Other boswellic acids include the keto-boswellic acids and their acetyl counterparts. Boswellic acids are produced by plants of the genus *Boswellia.* Like many other terpenes, boswellic acids appear in the resin of the plant that exudes them, and boswellic acids may be extracted from the trunks of e.g., *Boswellia Serrata, Boswellia carterii,* and various plants of the same genus. It is estimated that boswellic acids make up 30% of the resin of *Boswellia serrata.* Extracts of *Boswellia Serrata* contains various derivatives of boswellic acid including β-boswellic acid, acetyl-β-boswellic acid, 11-keto-β-boswellic acid and acetyl-11-keto-β-boswellic acid. Boswellic acids are known to exhibit anti-inflammatory behaviour.

WO2006/022762 describes a topical cream comprising 5% Boswellin^{®} and discloses the treatment of psoriasis patients with e.g., the disclosed topical cream applied trice daily for 150 days.

US 2011/0165136 describes a skin care composition for treatment of psoriasis comprising 0.3 - 1 % Boswellia extract.

### SUMMARY OF THE INVENTION

It is an object of the present disclosure to provide an improved skin care composition for treatment of Psoriasis.

It is a further object of the present disclosure to provide an improved method of treatment of psoriasis.

The present inventors surprisingly found that boswellic acids and vitamin B12 provided synergistical effects in the treatment of psoriasis, in particular plaque psoriasis. The inventors believe that the boswellic acids are effective in supressing the anti-inflammatory response resulting in psoriasis, while vitamin B12 is believed to decrease the content of NO (nitrogen oxide), which acts as a tissue skin irritant.

Thus, in a first aspect, the present invention relates to a skin care composition for the treatment of psoriasis comprising 1-10% boswellic acids and 0.02-2% vitamin B12 or derivatives thereof.

In a preferred embodiment, the compositions comprise 1-9%, such as 1-8%, such as 1-7%, such as 1-6%, such as 1-5%, such as 1-4%, such as 1-3%, such as 2.5% boswellic acids. In another embodiment, the compositions comprise 1.5-10%, such as 1.75-10%, such as 2-10%, such as 2.5% boswellic acids. In another embodiment, the compositions comprise 1.5-7%, such as 1.75-4.5%, such as 2-3%, such as 2.5% boswellic acids.

In another preferred embodiment, the compositions comprise 0.03-2%, such as 0.04-2%, such as 0.05-2%, such as 0.06-2%, such as 0.07% vitamin B12 or a derivative thereof. In another embodiment, the compositions comprise 0.03-1.5%, such as 0.03-1.2%, such as 0.03-1%, such as 0.03-0.9%, such as 0.03-0.8%, such as 0.07% vitamin B12 or a derivative thereof. In another embodiment, the compositions comprise 0.03-1%, such as 0.04-0.5%, such as 0.05-0.4%, such as 0.06-0.5%, such as 0.06-0.1%, such as 0.07% vitamin B12 or a derivative thereof.

Thus, in a preferred embodiment, the compositions comprise 1.5-7%, such as 1.75-4.5%, such as 2-3%, such as 2.5% Boswellic acids and 0.03-1%, such as 0.04-0.5%, such as 0.05-0.4%, such as 0.06-0.5%, such as 0.06-0.1%, such as 0.07% vitamin B12 or a derivative thereof.

Preferably, the boswellic acids are derived from extracts of Boswellia. Boswellia species are moderate-sized flowering plants, including both trees and shrubs, and are native to tropical regions of Africa and Asia. The four main species of Boswellia are *B. sacra* (synonyms *B. carterii* and B. *bhaw-dajiana*), *B. frereana, B. papyrifera,* and *B. serrata,* and each type of resin extract is available in various grades. The grades depend, e.g., on the time of harvesting. Boswellia extracts are available from numerous commercial sources, and the extract used in the preparation of the composition according to example 1 of the present disclosure was purchased from Shaanxi Yuantai Biological Technology Co., Ltd. This Boswellia extract was described by the manufacturer as being the gum resin extract exuded after cuts on the trunks of the olive family plant *Boswellia carterii* and various plants of the same genus. The extract used contained more than 95% boswellic acids.

Thus, in a preferred embodiment of the present invention, the boswellic acids are derived from extracts of plants of the genus *Boswellia.* Preferably, the main part of the boswellic acids is derived from extracts of plants of *Boswellia sacra* (*Boswellia carterii*).

The inventors further found that carbamide contributed further to the therapeutic activity of the composition. Carbamide (urea) is an organic compound with chemical formula CO(NH₂)₂. Carbamide is typically used in topical creams as a moisturising and peeling agent. In the present context, it is believed that carbamide act keratolytically and enhance penetration of both the boswellic acids and vitamin B12 through the top layers of the skin.

Accordingly, in a second aspect, the present invention relates to a composition comprising boswellic acids, carbamide and vitamin B12, or a derivative thereof.

More specifically, in a second aspect, the present invention relates to a skin care composition for treatment of psoriasis characterised by comprising 2-20% carbamide, 1-10% boswellic acids and 0.02-2% vitamin B12 or a derivative thereof.

In a preferred embodiment, the compositions according to the present invention comprises 2-20%, such as 5-15%, such as 5-14%, such as 5-13%, such as 5-12%, such as 5-11%, such as 10% carbamide. In a preferred embodiment, the compositions according to the present invention comprises 6-20%, such as 7-20%, such as 8-20%, such as 9-20%, such as 10% carbamide. In a preferred embodiment, the compositions according to the present invention comprises 6-14%, such as 7-13%, such as 8-12%, such as 9-11%, such as 10% carbamide.

The compositions of the present invention are intended for topical administration, i.e., they are formulated to be suitable for topical administration. Preferably, the compositions of the present invention are in the form of creams, lotions, ointments, gels, emulsions, solutions, or dermal patches, wherein a cream is particularly preferred. Methods for the formulation of the compositions of the present invention as a cream, a lotion, an ointment, a gel, an emulsion, a solution, or a dermal patch are not particularly limited and are known in the art.

Thus, the composition of the present invention further comprises one or more components selected from the group consisting of pharmaceutically and/or cosmetically acceptable excipients, solvents, preservatives, antioxidants, moisturizing agents, thickening agents, gelling agents, waxes, surfactants, emollients, emulsifiers, oil bases, ointment bases, penetration enhancers, solubilizers, softeners, chelating agents, fragrances, and buffering agents. Respective components are not particularly limited and are known in the art.

A particular example of a pharmaceutically and/or cosmetically acceptable solvent is water.

In addition to the active ingredients (boswellic acids, vitamin B12 or derivatives thereof and optionally carbamide), the compositions according to the present invention preferably comprise 20-40% emollients. Preferred emollients are selected among almond Oil (*Prunus Amygdalas Du*/*cis*)*,* Jojoba seed oil (*Simmondsia chinensis),* Caprylic/Capric Triglyceride and Paraffinum liquidum.

Further, the compositions according to the present invention preferably comprise 1-10% emulsifiers. Preferred emulsifiers are selected among Cetearyl alcohol, Myristyl Alcohol, Cetyl Alcohol, Glyceryl Stearate, PEG-75 Stearate, Ceteth-20, Steareth-20.

Further, the compositions according to the present invention preferably comprise 0-10% moisturising agents. Preferred moisturising agents are selected among Shea Butter (*Butyrospermum Parkii*).

Further, the compositions according to the present invention preferably comprise 0-2% preservatives. Preferred preservatives are selected among sodium benzoate, potassium benzoate and phenoxyethanol.

Further, the compositions according to the present invention preferably comprises 0-10% softeners. A preferred softeners is glycerine.

Further, the compositions according to the present invention preferably comprise 0-10% waxes. Preferred waxes are selected among Rhus succedanea fruit wax and Carnauba wax.

Further, the compositions according to the present invention preferably comprise 0-10% antioxidants. Preferred antioxidants are preferably selected among Tocopherol.

Further, the compositions according to the present invention preferably comprises 0-10% thickening agents. A preferred thickening agent is Kahlgum.

Further, the compositions according to the present invention preferably comprise 20-60% water. Thus, in a preferred embodiment, in addition to the active ingredients (boswellic acids, vitamin B12 or derivatives thereof and optionally carbamide), the composition of the present invention preferably comprises additional components in the following amounts:

**Table 1**

| **Function** | **%** |
|---|---|
| Emollients | 20-40% |
| Emulsifiers | 1-10% |
| Moisturizing agents | 0-10% |
| Preservatives | 0-2% |
| Softeners | 0-10% |
| Wax | 0-10% |
| Antioxidants | 0-10% |
| Thickening agents | 0-10% |
| Water | 20-60% |

As evidenced in the examples below, the compositions according to the invention are surprisingly efficient for the treatment of psoriasis. In particular, the significant reduction of itching and the significant reduction in the presence of scales show that the compositions are surprisingly efficient for the treatment of plaque psoriasis.

Further, the compositions may be used in general for reducing the presence of scales on human skin.

Further, the compositions may be used in general for reducing the itching of human skin.

Further, the present disclosure relates to a method of treatment of psoriasis, the method comprising applying the composition according to the invention to skin areas in need of treatment. In particular, it was observed that application of the composition twice daily showed significant benefits in many patients already after only 3 weeks of treatment and almost complete relief after only 4 weeks of treatment twice daily.

The disclosure has been described with reference to a preferred embodiment. However, the scope of the invention is not limited to the illustrated embodiment, and alterations and modifications can be carried out without deviating from the scope of the invention.

### EXAMPLES

### Example 1

A skincare composition comparison of the ingredients listed in Table 2 below was produced as follows:
Carbamide and water is mixed 24 hours before use. Almond oil, Rhus succedanea fruit wax, Jojoba oil, Cetearyl alcohol, Myristyl alcohol, Carnauba wax, Tocopherol, Paraffinum liquidum, Cetyl alcohol, Glycerol stearate, PEG-75 Stearate, Ceteth-20, Steareth-20 and Kahlgum are mixed and heated to 75°C providing a fatty phase. Glycerin, Water, Sodium benzoate and Potassium sorbate is mixed whereafter cyanocobalamin dissolved in water is added. The mixture is heated to 75°C providing a water phase. The fatty phase is transferred to the water phase and the resulting mixture is emulsified. After cooling to 35°C the Boswellic acids and Carbamide are added. pH is adjusted to 5.0-5.5 with citric acid.

**Table 2: Ingredient list**

| **INCI name** | **Function** | **%** |
|---|---|---|
| Almond Oil (Prunus Amygdalas Dulcis) | Emollient | 10 |
| Shea Butter (Butyrospermum Parkii) | Moisturizing agent | 4 |
| Rhus succedanea fruit wax | Wax | 4 |
| Jojoba (Simmondsia chinensis) seed oil | Emollient | |
| Cetearyl alcohol | Nonionic surfactant, emusifier | |
| Myristyl Alcohol | Emulsifier | |
| Caprylic/ Capric Triglyceride | Emollient | |
| Carnauba (Copernicia Cerifera Cera) | Wax | |
| Tocopherol | Antioxidant | |
| Paraffinum liquidum | Emollient | 20 |
| Cetyl Alcohol | Emulsifier | 3 |
| Glyceryl Stearate | Emulsifier | |
| PEG-75 Stearate | Emulsifier | |
| Ceteth-20 | Emulsifier | |
| Steareth-20 | Emulsifier | |
| Glycerin | Softener | 3 |
| Kahlgum | Thickening agent | 0.2 |
| Water | | 44.73 |
| Sodium benzoate | Preservative | 1.00 |
| Potassium sorbate | Preservative | |
| Cyanocobalamin | Active ingredient | 0.07 |
| Carbamide (Urea) | Active ingredient | 10 |
| Boswellic acids | Active ingredient | 2.5 |
| Citric acid | pH ajustment | |
| | | **100** |

### Example 2

The skincare composition provided in example 1 was delivered under confidentiality to 24 test persons all suffering from moderate to severe psoriasis. The test persons were aged 21-73 years (median 39 years) and were instructed to apply the composition according to Example 1 to only one selected skin part in need of treatment, leaving other parts of their skin untreated (or treated according to the normal procedure of the respective test person). Test persons were also instructed to continue any medical treatment which the test person was currently employing, such as any topical treatment on the rest of their skin parts in need of treatment. Test persons were instructed to apply the composition once or twice per day (dependent on the severity of the psoriasis) for 4 weeks and to apply the composition as a thin layer. Test persons were instructed to photograph and document their skin before and after the 4-week treatment. Each test person completed a questionnaire following the 4-week treatment.

**Table 3: Age of the test persons**

| **Test person #** | **Age (years)** |
|---|---|
| 1 | 48 |
| 2 | 74 |
| 3 | 39 |
| 4 | 30 |
| 5 | 38 |
| 6 | 25 |
| 7 | 21 |
| 8 | 56 |
| 9 | 70 |
| 10 | 32 |
| 11 | 31 |
| 12 | 63 |
| 13 | 65 |
| 14 | 78 |
| 15 | 35 |
| 16 | 51 |
| 17 | 33 |
| 18 | 57 |
| 19 | 55 |
| 20 | 42 |
| 21 | 39 |
| 22 | 28 |
| 23 | 32 |
| 24 | 52 |

### General results

23 of the 24 test persons showed significant ameliorations of the symptoms or even complete remission, and only one test person would not recommend the composition to other persons suffering from psoriasis. None of the test persons reported any negative side effects. The test persons had in general been suffering significantly from their respective diseases irrespective of other treatments regimens being followed previously.

### Questionnaire results

Each test person completed an electronic questionnaire following the 4-week treatment. The questionnaire included the below questions as well as questions related to other treatments on the market.
*How would you characterize your skin BEFORE application of the composition? (The test persons were allowed to select multiple answers)*

| **Red** | **Irritated** | **Raised skin** | **Itchy** | **Eczema present** | **Scaly** |
|---|---|---|---|---|---|
| 66.67% | 58.33% | 45.83% | 70.83% | 45.83% | 100% |

*How would you characterize your skin AFTER application of the composition? (The test persons were allowed to select multiple answers)*

| **Smooth** | **Pliable** | **No raised skin** | **Not itchy** | **Uniform** | **Scaly** |
|---|---|---|---|---|---|
| 33.33% | 33.33% | 37.50% | 70.83% | 8.33% | 12.50% |

*Please describe how much your skin itched BEFORE application of the composition?*

| **0 = no itching** | **1 = low level of itching** | **2 = moderate level of itchinq** | **3 = constant itching** | **4 = significant itching** | **5 = unbearable itching** | **Average** |
|---|---|---|---|---|---|---|
| 4.17% | 25.00% | 12.50% | 20.83% | 33.33% | 4.17% | **3.67** |

*Please describe how much your skin itched AFTER application of the composition?*

| **0 = no itching** | **1 = low level of itching** | **2 = moderate level of itchinq** | **3 = constant itching** | **4 = significant itching** | **5 = unbearable itching** | **Average** |
|---|---|---|---|---|---|---|
| 41.67% | 33.33% | 20.83% | 0% | 4.17% | 0% | **1.92** |

*Please describe how red your skin was BEFORE application of the composition?*

| **0 = not red** | **1 = slightly red** | **2 = moderately red** | **3 = red** | **4 = very red** | **5 = extremely red** | **Average** |
|---|---|---|---|---|---|---|
| 4.17% | 12.50% | 20.83% | 12.50% | 45.83% | 4.17% | **3.96** |

*Please describe how red your skin was AFTER application of the composition?*

| **0 = not red** | **1 = slightly red** | **2 = moderately red** | **3 = red** | **4 = very red** | **5 = extremely red** | **Average** |
|---|---|---|---|---|---|---|
| 0% | 33.33% | 25.00% | 20.83% | 20.83% | 0% | **3.29** |

*Would you recommend the composition to others for treatment of Psoriasis?*

| **Yes** | **No** | **Do not know** | **No response** |
|---|---|---|---|
| 70.73% | 4.17% | 16.67% | 8.33% |

*Was your quality of life reduced due to your Psoriasis BEFORE application of the composition?*

| **Yes** | **No** | **Do not know** | **No response** |
|---|---|---|---|
| 75.00% | 20.83% | 4.17% | 0% |

*Has your quality of life been improved AFTER application of the composition due to skin relief?*

| **Yes** | **No** | **Do not know** | **No response** |
|---|---|---|---|
| 41.67% | 29.17% | 35.50% | 0% |

### Case reports

Patient #2:
A 74-year-old female suffering from psoriasis. The disease started on the elbows at the age of 54 and has now spread to other body parts such as the ears, neck and knees.

She is currently treated with Enstilar^{®} and experiences scaly, red eczema with significant itching but no pain.

After application of the composition according to example 1 once daily to the elbows, the scale and the red eczema diminished significantly. After 6 weeks of treatment, both disappeared almost entirely, and she no longer uses Enstilar^{®}.

Patient #8:
A 56-year-old female suffering from severe psoriasis. The disease started at the age of 10 and has now spread to 50% of the skin surface.

She is currently treated with Enstilar^{®} and experiences scaly, red eczema with significant itching and pain.

After application of the composition according to example 1, the scale and the red eczema diminished significantly. The scale, itching and pain have now disappeared, and she wishes to receive more of the composition according to example 1 and consider it to have had a significant effect.

Patient #11:
A 31-year-old male suffering from severe psoriasis. The disease started on the arms at the age of 14 and disappeared after being treated with hormone cream. At the age of 21, the disease reappeared and has now significantly spread to the knees, elbows, and legs. He has tried a range of medications without any significant effect. He never wears shorts and does not wish to expose the diseased skin parts to the public.

He experiences scaly, red eczema. The skin is painful when stretched and has a tendency to bleed from small scratches caused by the disease.

He was sceptical with respect to the efficacy of the composition according to example 1. However, after only one week of treatment with the composition according to example 1, he experienced that the pain and the itching disappeared entirely. He now has a much better quality of life.

Patient #16:
A 51-year-old female suffering from severe psoriasis. The disease started on the arms and legs at the age of 21 and has now spread to 80% of the skin surface, including the face. She is tired and is currently being diagnosed with psoriatic arthritis.

She has tried a range of treatments and is currently treated with Enstilar^{®}. She experiences scaly, red eczema with significant itching and pain. She has also been offered climate treatment.

After application of the composition according to example 1 twice daily, the scale and the red eczema diminished significantly. The skin is now moist and softer, and the itching and pain have disappeared. She now combines treatment with the composition according to example 1 and treatment (once every 3-4 days) with Enstilar^{®}. She describes the composition according to example 1 as the first cream ever to really have a significant effect.

## Claims

1. A skin care composition comprising 1-10% boswellic acids and 0.02-2% vitamin B12 or a derivative thereof.

2. A skin care composition according to claim 1 comprising 0.03-1%, such as 0.04-0.5%, such as 0.05-0.4%, such as 0.06-0.5%, such as 0.06-0.1%, such as 0.07% vitamin B12 or a derivative thereof.

3. A skin care composition according to claim 1 or 2 comprising 1.5-7%, such as 1.75-4.5%, such as 2-3%, such as 2.5% boswellic acids.

4. A skin care composition according to any of the preceding claims comprising 2-3%, such as 2.5% boswellic acids and 0.06-0.1%, such as 0.07%, vitamin B12 or a derivative thereof.

5. A skin care composition according to any of the preceding claims, wherein the boswellic acids are derived from extracts of Boswellia.

6. A skin care composition according to any of the preceding claims comprising 2-20%, such as 5-15%, such as 5-14%, such as 5-13%, such as 5-12%, such as 5-11%, such as 10% carbamide.

7. A skin care composition according to any of the preceding claims comprising 2-20% carbamide, 1-10% boswellic acids and 0.02-2% vitamin B12 or a derivative thereof.

8. A skin care composition according to any of the preceding claims for use in the treatment of psoriasis.

9. A skin care composition according to claim 8, wherein the psoriasis is plaque psoriasis.

10. A skin care composition according to any of the preceding claims wherein the composition is a cream.

11. Use of a skin care composition according to any of the preceding claims for treatment of psoriasis.

12. A method for the treatment of psoriasis, the method comprising applying the composition according to any of the preceding claims 1-10 to skin areas in need of treatment.
